(19) **Europäisches Patentamt**
**European Patent Office**
**Office européen des brevets**

(11) Veröffentlichungsnummer: **0 073 051**
**A2**

(12) # EUROPÄISCHE PATENTANMELDUNG

(21) Anmeldenummer: 82107692.4

(22) Anmeldetag: 23.08.82

(51) Int. Cl.³: **C 01 B 33/28**
C 01 B 33/158, B 01 J 29/06
B 01 J 21/08, C 07 C 11/02
C 07 C 1/20

(30) Priorität: 26.08.81 DE 3133747

(43) Veröffentlichungstag der Anmeldung:
02.03.83 Patentblatt 83/9

(84) Benannte Vertragsstaaten:
BE DE FR GB IT NL

(71) Anmelder: HOECHST AKTIENGESELLSCHAFT
Postfach 80 03 20
D-6230 Frankfurt/Main 80(DE)

(72) Erfinder: Litterer, Heinz, Dr.
Albert-Schweitzer-Allee 39
D-6200 Wiesbaden(DE)

(72) Erfinder: Wunder, Friedrich, Dr.
Jahnstrasse 46
D-6093 Flörsheim am Main(DE)

(72) Erfinder: Leupold, Ernst Ingo, Dr.
Am Zäunefeld 15
D-6392 Neu-Anspach(DE)

(72) Erfinder: Baltes, Herbert, Dr.
Johannesallee 24
D-6230 Frankfurt am Main 80(DE)

(54) Alumosilikate und Kieselgele mit geringem Gehalt an Übergangselementen, Verfahren zu deren Herstellung und ihre Verwendung.

(57) Die vorliegende Erfindung betrifft Alumosilikate und Kieselgele mit geringem Gehalt an Übergangselementen, ein Verfahren zu deren Herstellung sowie deren Verwendung als Katalysatoren oder Katalysatorträger. Die Herstellung erfolgt durch Behandlung der Alumosilikate bzw. Kieselgele mit Lösungen von Komplexbildnern bei 70 bis 150°C. Eine Verwendungsmöglichkeit der Alumosilikate als Katalysatoren ist ihre Einsatz bei der Herstellung niederer Olefine aus Methanol.

EP 0 073 051 A2

HOECHST AKTIENGESELLSCHAFT          Dr.MA/AK      HOE 81/F 216

Alumosilikate und Kieselgele mit geringem Gehalt an Übergangselementen, Verfahren zu deren Herstellung und ihre Verwendung.

Die vorliegende Erfindung betrifft Alumosilikate und Kieselgele mit geringem Gehalt an Übergangselementen, ein Verfahren zu deren Herstellung sowie deren Verwendung als Katalysatoren oder Katalysatorträger.

Als Übergangselemente kommen dabei vor allem Cu, Ni, V, Ti, Zr, Co und insbesondere Fe in Frage. Diese Elemente können als Metalle oder Verbindungen vorliegen.

Aus der Literatur ist bekannt, daß Übergangselemente, insbesondere Eisen, bei zahlreichen heterogen katalysierten Stoffumwandlungen stören, und zwar vor allem durch Selektivitätsminderung. (Furimski, E., Erdöl und Kohle (1979) 32 (8), 383).

Natürliche Zeolithe enthalten häufig Übergangselemente. Andere Alumosilikate und Kieselgele werden oftmals bei ihrer Herstellung mit Übergangselementen verunreinigt.

Für die Entfernung von Übergangselementen aus Kieselgelen und säurebeständigen Alumosilikaten sind Reinigungsverfahren mit verdünnten Mineralsäuren bekannt

Jedoch verursachen diese Verfahren eine häufig störende Acidität und sind nicht sehr effektiv. Für wenig säurebeständige Alumosilikate sind sie ganz ungeeignet.

Es ist bisher kein wirtschaftliches Verfahren zur Entfernung von Übergangselementen aus Zeolithen oder sonstigen Alumosilikaten mit Komplexbildnern ohne Änderung des Al-Gehalts bekannt. Aus DE-OS 2 928 922 ist ein Verfahren zur Herstellung von $C_2$-$C_4$ - Olefinen aus Methanol und/oder Dimethylether in Gegenwart von Wasser an einem Mangan enthaltenden Alumosilikat-Katalysator bekannt, das dadurch gekennzeichnet ist, daß der Katalysator mit einer Lösung von Ethylendiamintetraessigsäure oder Weinsäure mit einem pH von 3 bis 7 ausgewaschen wird. Die Temperatur der Lösung liegt vorzugsweise zwischen 0 und 50°C. Dabei kann Mangan vor oder nach dieser Wäsche auf den Träger aufgebracht werden. Daraus folgt, daß das Übergangselement Mangan bei dieser Wäsche weder entfernt wird, noch entfernt werden soll. Zweck dieser Maßnahmen ist nämlich die Bereitstellung eines manganhaltigen Katalysators für die Olefinherstellung aus Methanol und/oder Dimethylether, der gegen größere Mengen Wasser stabil ist.

Ein Gegenstand der vorliegenden Erfindung sind Alumosilikate oder Kieselgele mit geringem Gehalt an Übergangselementen, dadurch gekennzeichnet, daß die Alumosilikate bzw. Kieselgele bei Temperaturen von 70 bis 150°C mit Lösungen von Komplexbildnern behandelt wurden.

Ein weiterer Gegenstand der Erfindung ist ein Verfahren zur Herstellung von Alumosilikaten oder Kieselgelen mit gerigem Gehalt an Übergangselementen, dadurch gekennzeichnet, daß man die Alumosilikate oder Kieselgele bei Temperaturen von 70 bis 150°C mit Lösungen von Komplexbildnern behandelt. Dabei werden die Übergangselemente nahezu quantitativ ent-

fernt; dies gilt insbesondere für die häufigste Verunreinigung, nämlich Eisen. Vorzugsweise arbeitet man bei
Temperaturen von 80 bis 120°C, insbesondere bei 90 bis
110°C.

Ein weiterer Gegenstand der Erfindung ist die Verwendung
von wie erwähnt behandelten Alumosilikaten bzw. Kieselgelen als Katalysatoren bzw. Katalysatorträger bei der Umsetzung einer organischen Charge, dadurch gekennzeichnet,
daß man die Charge unter Umsetzungsbedingungen mit den
Alumosilikaten oder Kieselgelen, ggf. nach Dotierung mit
einem katalytisch wirksamen Agens, in Kontakt bringt. Die
Zeitdauer der Behandlung mit Lösungen von Komplexbildnern
beträgt i.allg. 10 bis 150 Stunden, vorzugsweise 40 bis
120 Stunden. Bei besonders stabilen Zeolith-Typen, wie
Mordenit und Ferrierit, kann die Zeitdauer auch noch
wesentlich länger sein, was allerdings nicht notwendig
ist.

Der Befund, daß nach dem erfindungsgemäßen Verfahren mit
Übergangselementen verunreinigte natürliche und synthetische Zeolithe sowie andere Alumosilikate in wirtschaftlich vertretbaren Zeiten nahezu quantitativ gereinigt
werden können, ohne daß größere Anteile des Gerüstaluminiums entfernt werden, ist außerordentlich überraschend.
Sogar natürliche kleinporige Zeolithe mit Porenöffnungen
von weniger als 4.5 Å mit Komplexbildnern, deren kinetischer Moleküldurchmesser wesentlich größer als 4.5 Å ist,
werden nahezu quantitativ von Eisenverunreinigungen befreit. Bei der Entfernung von Eisen bzw. anderen Übergangselementen aus Kieselgelen wurden überraschenderweise
bessere Resultate als mit verdünnten Mineralsäuren erzielt.

Geeignete Komplexbildner sind chelatisierende Carbonsäuren,
wie Hydroxycarbonsäuren (insbesondere Weinsäure und Zi-

tronensäure) oder Aminocarbonsäuren (insbesondere Ethylendiamintetraessigsäure, Hydroxyethylendiamintetraessigsäure und Nitrilotriessigsäure). Weiter sind geeignet 1.3—Diketone (insbesondere Acetylaceton und Trifluoracetylaceton), Aminoalkohole, Aminophenole, Phosphorsäuren, Phosphonsäuren (insbesondere Ethylendiamintetramethylenphosphonsäure und Nitrilotrimethylenphosphonsäure). Auch Derivate der genannten Verbindungen sind geeignet. Anorganische Cyanide und Rhodamide können ebenso eingesetzt werden. Vorzugsweise verwendet man chelatisierende Carbon-, Phosphor- oder Phosphonsäuren oder deren Derivate. Besonders geeignet sind die Salze der Ethylendiamintetraessigsäure und der Ethylendiamintetramethylenphosphonsäure.

Nach dem erfindungsgemäßen Verfahren können Übergangselemente wie Cu, Ni, V, Ti, Zr, Co und insbesondere Fe praktisch vollständig entfernt werden.

Als Lösemittel können Wasser, organische Lösemittel wie Methanol oder Formamid, Gemische organischer Lösemittel oder wasserhaltige organische Lösemittel oder Lösemittelgemische eingesetzt werden. Vorzugsweise verwendet man Wasser oder wasserhaltige organische Lösemittel oder wasserhaltige organische Lösemittelgemische. Insbesondere bei höheren Reaktionstemperaturen sind auch hochsiedende Alkohole oder Carbonsäuren einsetzbar.

Das erfindungsgemäße Verfahren wird im allgemeinen unter Atmosphärendruck oder dem jeweiligen Dampfdruck des Lösemittels durchgeführt, es ist jedoch auch möglich, durch Zusatz von Inertgasen den Druckbereich speziellen Gegebenheiten anzupassen.

0073051

Der Konzentrationsbereich für den organischen Komplexbildner liegt im allgemeinen zwischen 3 und 40 Gew.-%, bezogen auf das jeweilige Lösemittel,vorzugsweise zwischen
7 und 30 Gew.-%.

Das Verfahren kann in den üblichen Reaktionsbehältern sowohl kontinuierlich als auch diskontinuierlich durchgeführt werden.

Das Verhältnis der Volumina von Komplexbildner-Lösung zu
Alumosilikat oder Kieselgel liegt im allgemeinen im Bereich von 0.3 bis 10.

Die erfindungsgemäß von Übergangselementen befreiten Alumosilikate bzw. Kieselgele können
nach Entfernung von Lösemittel und Komplexbildner als
Katalysatoren oder Katalysatorträger verwendet werden.

Insbesondere sei hier erwähnt die Umwandlung von Methanol
in kurzkettige Olefine an kleinporigen, natürlichen Zeolithen, das selektive Cracken (Selectoforming) an natürlichen Zeolithen vom Erionit- und Ferrierittyp, sowie die
selektive Alkylierung von Benzol und dessen Derivaten an
natürlichen Ferrierit-Katalysatoren.

Die Erfindung soll durch die folgenden Beispiele erläutert werden, wobei die Beispiele aber in keiner Weise
einschränkend sein sollen. Die Versuche werden in einem
von außen beheizbaren, kontinuierlich betriebenen Rührkessel durchgeführt.

Beispiel 1

Man läßt 200 g eines natürlichen Chabasit-Erionit-Gemisches, dessen Eisengehalt 2.6 Gew.-% beträgt, mit 800 g einer 10 Gew.-%igen wäßrigen Lösung der Ethylendiamintetraessigsäure 96 Stunden bei 140°C reagieren. Nach Beendigung der Reaktion liegt der Eisengehalt unter 0.2 Gew.-%. Das Silizium-Aluminium-Verhältnis dieses Zeolithgemischs hat sich nur geringfügig von 3.90 auf 4.04 geändert.

Beispiel 2

100 g eines natürlichen Chabasit-Erionit-Gemischs mit einem Eisengehalt von 1.8 Gew.% werden mit 1000 g einer 5 Gew.-%igen wäßrigen Lösung des Dinatriumsalzes der Ethylendiamintetraessigsäure versetzt und 120 Stunden unter Rühren auf 70°C erhitzt.

Nach Beendigung der Reaktion hat sich das Silizium-Aluminium-Verhältnis von 4.0 auf 4.15 geändert. Der Eisengehalt des Zeolithgemischs beträgt nur noch 0.3 Gew.-%.

Beispiel 3

100 g eines natürlichen Chabasits mit einem Si/Al-Verhältnis von 3.03 und einem Eisengehalt von 2.8 Gew.-% werden mit 1000 g einer 20 Gew.-%igen wäßrigen Lösung des Natriumsalzes der Ethylendiamintetraessigsäure 48 Stunden unter Rückflußbedingungen behandelt.

Der Eisengehalt des Zeolithen beträgt nur noch 0.3 Gew.-%. Das Silizium-Aluminiumverhältnis hat von 3.03 auf 3.30 zugenommen.

## Beispiel 4

100 g eines eisenhaltigen natürlichen Ferrierits werden mit 1000 ml einer 10 Gew.-%igen wäßrigen Lösung des Na-Salzes der Ethylendiamintetramethylenphosphonsäure versetzt und 72 Stunden unter Rückflußbedingungen behandelt. Nach Beendigung der Reaktion ist der Eisengehalt des Alumosilikats von 2.4 % auf 0.2 % abgefallen.

Das Silizium-Aluminiumverhältnis hat sich von 4.95 auf 5.05 erhöht.

## Beispiel 5

Dieses Beispiel soll die selektivitätsverbessernde Wirkung des erfindungsgemäßen Verfahrens auf einen Zeolith-Katalysator für die Umsetzung von Methanol zu niederen Olefinen zeigen.

In einen senkrecht angeordneten, elektrisch beheizten Rohrreaktor von 1 m Länge, der mit 250 ml Katalysator gefüllt ist, dosiert man stündlich 520 ml 50 Gew.-%iges wässriges Methanol bei einer Temperatur von 400°C. Das Reaktionsgemisch wird abgekühlt, und nach Abtrennung der kondensierbaren Anteile wird die gasförmige Phase analysiert. Dieser Versuch wurde mit einem erfindungsgemäß behandelten und einem unbehandelten Zeolith-Katalysator durchgeführt:

a) Mit einem nach dem erfindungsgemäßen Verfahren behandelten Chabasit-Erionit-Gemisch, bei dem der Eisengehalt auf 0.2 Gew.-% reduziert wurde, ergab sich eine Selektivität an $C_2$-$C_4$-Olefinen von 83 %.

b) Bei Einsatz eines unbehandelten Chabasit-Erionit-Gemisches mit einem Eisengehalt von 2.9 Gew.-% wird eine Selektivität an $C_2$-$C_4$-Olefinen von 61 % erzielt.

BAD ORIGINAL

1. Verfahren zur Herstellung von Alumosilikaten oder Kieselgelen mit geringem Gehalt an Übergangselementen, dadurch gekennzeichnet, daß man die Alumosilikate oder Kieselgele bei Temperaturen von 70 bis 150°C mit Lösungen von Komplexbildnern behandelt.

2. Verfahren nach Anspruch 1, dadurch gekennzeichnet, daß man als Komplexbildner chelatisierende Carbon-, Phosphor- oder Phosphonsäuren oder deren Derivate einsetzt.

3. Verfahren nach Anspruch 1, dadurch gekennzeichnet, daß man Lösungen von Komplexbildnern in Wasser oder wasserhaltigen organischen Lösemitteln oder wasserhaltigen organischen Lösemittelgemischen verwendet.

4. Alumosilikate oder Kieselgele mit geringem Gehalt an Übergangselementen, dadurch gekennzeichnet, daß die Alumosilikate bzw. Kieselgele bei Temperaturen von 70 bis 150°C mit Lösungen von Komplexbildnern behandelt wurden.

5. Verwendung von Alumosilikaten oder Kieselgelen gemäß Anspruch 4 als Katalysatoren bzw. Katalysatorträger bei der Umsetzung einer organischen Charge, dadurch gekennzeichnet, daß man die Charge unter Umsetzungsbedingungen mit den Alumosilikaten oder Kieselgelen, ggf. nach Dotierung mit einem katalytisch wirksamen Agens, in Kontakt bringt.

6. Verwendung von Alumosilikaten gemäß Anspruch 4 als Katalysator bei der Herstellung von $C_2$-$C_4$-Olefinen aus Methanol.